# FASCICULE DE BREVET EUROPEEN

(11) **EP 1 392 838 B1**
(45) Date de publication et mention de la délivrance du brevet: **31.01.2007**
(21) Numéro de dépôt: 02740846.7
(22) Date de dépôt: 31.05.2002
(51) Int. Cl.: C12N 15/86, A61K 48/00, C12N 7/04, C12N 5/10, C07K 14/145

(54) **PSEUDOTYPAGE DE VECTEURS HIV PAR DES ENVELOPPES DE VIRUS MOKOLA**
PSEUDOTYPIERUNG VON HIV VEKTOREN MIT MOKOLA VIRENHÜLLEN
PSEUDOTYPING OF HIV VECTORS WITH MOKOLA VIRUS ENVELOPES

(30) Priorité: 01.06.2001 FR 0107239
(43) Date de publication de la demande: 03.03.2004
(73) Titulaire: CENTRE NATIONAL DE LA RECHERCHE SCIENTIFIQUE, 75016 Paris Cédex (FR)
(72) Inventeur: SARKIS, Chamsy, F-75017 PARIS (FR); HE, Yi, F-75013 Paris (FR); SERGUERA, Che, F-75010 Paris (FR); DUFOUR, Noelle, F-91780 Mennecy (FR); MALLET, Jacques, F-75013 Paris (FR)
(74) Mandataire: Becker, Philippe
(86) Numéro de dépôt international: PCT/FR2002/001833
(87) Numéro de publication internationale: WO 2002/097104

(56) Documents cités:
- WO-A-99/61639
- AURICCHIO A; KOBINGER G; ANAND V; HILDINGER M; MAGUIRE A M; WILSON J M; BENNET J.: "Adeno-associated virus type 5 and ---pseudotyped lentiviral vectors as novel gene delivery agents for the retina." IOVS, vol. 42, no. 1, 15 mars 2001 (2001-03-15), page S125 XP001058954 & Annual Meeting of the Association for Research in Vision and Ophthalmology; Fort Lauderdale, Florida, USA; April 29-May 04, 2001
- MITROPHANOUS K A ET AL: "STABLE GENE TRANSFER TO THE NERVOUS SYSTEM USING A NON-PRIMATE LENTIVIRAL VECTOR" GENE THERAPY, MACMILLAN PRESS LTD., BASINGSTOKE, GB, vol. 6, no. 11, 1999, pages 1808-1818, XP000914884 ISSN: 0969-7128
- MOCHIZUKI HIDEKI; SCHWARTZ JOAN P; TANAKA KOICHI; BRADY ROSCOE O; REISER JAKOB: "HIGH-TITER HUMAN IMMUNODEFICIENCY VIRUS TYPE 1-BASED VECTOR SYSTEMS FOR GENE DELIVERY INTO NONDIVIDING CELLS." JOURNAL OF VIROLOGY, vol. 72, no. 11, novembre 1998 (1998-11), pages 8873-8883, XP000783942
- ZENNOU V, SERGUERA C, SARKIS C, COLIN P, PERRET E, MALLET J, CHARNEAU P.: "The HIV-1 DNA flap stimulates HIV vector-mediated cell transduction in the brain." NAT BIOTECHNOL. 2001 MAY;19(5):446-50., XP001058913
- DESMARIS N, BOSCH A, SALAUN C, PETIT C, PREVOST MC, TORDO N, PERRIN P, SCHWARTZ O, DE ROCQUIGNY H, HEARD JM: "Production and neurotropism of lentivirus vectors pseudotyped with lyssavirus envelope glycoproteins." MOL THER. 2001 AUG;4(2):149-56., XP001058924
- MAZARAKIS NICHOLAS D. ET AL.: "Rabies virus glycoprotein pseudotyping of lentiviral vectors enables retrograde axonal transport and access to the nervous system after peripheral delivery." HUMAN MOLECULAR GENETICS, vol. 10, no. 19, 15 septembre 2001 (2001-09-15), pages 2109-2121, XP001058914
- AZZOUZ M. ET AL.: " Gene transfer to the nervous system using Equine Infectious Anaemia Virus based lentiviral vectors." SOCIETY FOR NEUROSCIENCE ABSTRACTS, XP001058953 & 31st Annual Meeting of the Society for Neuroscience; San Diego, California, USA; November 10-15, 2001

## Description

Le champ technique de la présente invention se rapporte au transfert de gènes dans le système nerveux au moyen de virus pseudotypés, et notamment au moyen de vecteurs lentiviraux défectifs pseudotypés avec une enveloppe de lyssavirus. Le but est le ciblage d'une population ou d'une sous-population cellulaire du système nerveux. La présente demande décrit des méthodes et compositions destinées au transfert sélectif de gènes cibles dans des populations de cellules nerveuses particulières, notamment dans les astrocytes, *in vitro*, *ex vivo* et *in vivo*, et discute également leur utilisation dans le traitement de désordres du système nerveux central et/ou du système oculaire.

Le transfert de gènes dans le système nerveux présente de multiples applications, dans les domaines expérimentaux, thérapeutiques, de recherche, etc. Ainsi, ce transfert peut permettre la réalisation d'études de marquage, de toxicité, de qualité, la construction de modèles pathologiques, la restauration de déficits, l'expression de produits (e.g., protéines, ARNs, etc.) thérapeutiques, etc.
Différentes approches ont été envisagées dans l'art antérieur pour ce transfert, telles que l'emploi de vecteurs viraux (rétrovirus, Adéno-associated virus (AAV), adénovirus, etc.), l'injection de plasmides, la greffe de cellules, l'implantation de cellules encapsulées, etc. Chacune de ces approches présente des avantages et des inconvénients, en terme d'efficacité, de sécurité, d'utilisation industrielle, de sélectivité, de stabilité, etc.
Ainsi, l'utilisation de vecteurs viraux est avantageuse en terme d'efficacité de transfert, liée aux propriétés naturelles d'infection des virus. Parmi les virus utilisés, le demandeur s'est intéressé plus particulièrement aux rétrovirus.

Dans les premières étapes de l'infection, les rétrovirus délivrent leur corps nucléoprotéique dans le cytoplasme de la cellule cible. Se produit alors la transcription inverse du génome viral tandis que le corps se transforme en un complexe de préintégration. Le complexe doit atteindre le noyau pour permettre l'intégration de l'ADN viral dans les chromosomes de la cellule hôte.

De nombreux vecteurs rétroviraux dérivés d'oncorétrovirus permettent l'intégration d'un transgène dans le génome de cellules cibles mais ces vecteurs sont uniquement capables de transduire des cellules en division. Cette restriction limite leur utilisation au transfert de gène *ex vivo* ou aux organes dont les cellules sont mitotiquement actives.

Un moyen de contourner cet obstacle consiste à utiliser des vecteurs dérivés de lentivirus. Les lentivirus sont des rétrovirus complexes capables de s'intégrer dans le génome de cellules non mitotiquement actives. Ces virus comptent notamment parmi eux le virus de l'immunodéficience humaine de type 1 (HIV-1), le virus de l'immunodéficience humaine de type 2 (HIV-2), le virus de l'immunodéficience simienne (SIV), le virus de l'immunodéficience féline (FIV), le virus de l'immunodéficience bovine (BIV), le virus du VISNA-Maedi (VISNA), le virus de l'arthrite encéphalite caprine (CAEV) et le virus de l'anémie infectieuse équine (EIAV). Toutefois, des inconvénients liés à l'utilisation de lentivirus résident notamment dans l'absence de spécificité, les risques potentiels et les difficultés de production. Les inconvénients ont jusqu'à présent limité l'exploitation de ce type de vecteur pour le transfert de gènes *in vivo*.

Pour ce qui concerne la spécificité des vecteurs, l'art antérieur enseigne qu'un ciblage préférentiel, sinon spécifique, de certaines sous-populations de cellules neuronales est possible avec des vecteurs pseudotypés.

AURICCHIO et al. (IOVS, vol 42, no 1, 15 mars 2001, page S125) mentionne l'utilisation de vecteurs lentiviraux pseudotypés avec l'enveloppe du virus MOKOLA pour le transfert dans des cellules de l'épithélium pigmentaire rétinien suivant l'injection subrétinienne chez la souris à des fins de thérapie génique rétinienne.

MOCHIZUKI et al. (JOURNAL OF VIROLOGY, vol 72, no 11, novembre 1998, pages 8873-8883) décrit la production en culture cellulaire de vecteurs lentiviraux défectifs issus de HIV-1 pseudotypés avec les protéines enveloppes G du virus de la rage et du virus MOLOKA et enseigne une transduction de cellules HOS et Rat-2. Il n'est pas fait mention de la possibilité de transduire des astrocytes.

WO 99/61639 décrit l'utilisation de vecteurs lentiviraux défectifs dérivés du virus de l'immunodéficience humaine de type 1 (HIV-1) et du virus de l'anémie infectieuse équine (EIAV) pseudotypés avec la protéine enveloppe G du virus de la rage à des fins de thérapie génique et suggère que le tropisme des pseudotypes devrait être identique au tropisme dont dérive l'enveloppe. Il prévoit que le tropisme de rétrovirus pseudotypés avec la protéine rabique devrait être neuronal et cite les astrocytes sans toutefois fournir de données précises concernant le ciblage cellulaire des pseudotypes comme cellules cibles des vecteurs.

MITROPHANOUS K.A. et al.(Gene therapy, vol. 6, no. 11, 1999, pages 1808-1818) décrit l'utilisation de vecteur lentiviral défectif dérivé du virus de l'anémie infectieuse équine (EIAV) pseudotypé avec la protéine enveloppe G du virus de la rage pour le transfert de gène mais ne présente aucune donnée concernant le ciblage cellulaires des pseudotypes.

ZENNOU V, et al. (NAT BIOTECHNOL. 19(5) 2001, pages 446-50) montre que l'insertion d'une séquence flap dans des vecteurs lentiviraux dérivés de HIV améliore la transduction génique dans les cellules neurales, *ex vivo* et *in vivo* dans le cerveau de rat.

Il reste un besoin dans l'art de développer des vecteurs pour le ciblage d'astrocytes, ce qui permettrait une éventuelle thérapie de certaines maladies comme celle de Parkinson.

La présente invention apporte une solution à cet égard et fournit de nouveaux outils et vecteurs lentiviraux pseudotypés avec des enveloppes de virus Mokola pour le transfert sélectif de gènes dans les astrocytes du système nerveux, central in vivo, in vitro et ex vivo.

La présente invention décrit en outre un vecteur semblable pseudotypé avec la protéine G du virus de la rage, pouvant être utilisé pour le même but. Ce vecteur est décrit à des fins illustratives et ne fait pas partie de l'invention.

### Description détaillée de l'invention

La présente invention concerne le pseudotypage de vecteurs lentiviraux recombinants par une enveloppe de virus Mokola permettant le ciblage cellulaire sélectif *in vivo* dans les cellules gliales de type astrocytaire. Ces vecteurs viraux pseudotypés sont utiles pour le transfert et l'expression *in vivo* de séquences d'acides nucléiques au sein des astrocytes.

### Structure générale des vecteurs

Comme d'autres rétrovirus, les lentivirus possèdent des gènes *gag, pol* et env flanqués de deux séquences LTR (Long Terminal Repeat). Chacun de ces gènes code pour de nombreuses protéines qui sont initialement exprimées sous la forme d'un unique polypeptide précurseur. Le gène gag code les protéines de structure internes (capsides et nucleocapside). Le gène *pol* code la transcriptase inverse, l'integrase et la protease. Le gène *env* code la glycoprotéine d'enveloppe virale et contient en outre un élément RRE (Rev Responsive Element) agissant en *cis* responsable de l'export hors du noyau de l'ARN viral. Les séquences LTR 5' et 3' servent à promouvoir la transcription et la polyadénylation des ARN viraux. Le LTR contient toutes les autres séquences agissant en *cis* nécessaires à la réplication virale. Des séquences nécessaires à la transcription inverse du génome (site de liaison de l'amorce de l'ARNt) et à l'encapsidation de l'ARN viral dans des particules (site Ψ) sont adjacentes au LTR 5'. Si les séquences nécessaires à l'encapsidation (ou à l'empaquetage de l'ARN rétroviral dans les virions infectieux) sont absentes du génome viral, l'ARN génomique ne sera pas activement encapsidé.

La construction de vecteurs lentiviraux pour des applications de transfert de gènes a été décrite par exemple dans les brevets US 5,665,577, EP 386 882, US 5,981,276, US 6, 013, 516 ou encore dans la demande de brevet WO 99/58701.
Ces vecteurs comportent un génome lentiviral défectif, c'est-à-dire dans lequel l'un au moins des gènes *gag, pol* et *env* a été inactivé ou délété. Ceux-ci sont encapsidés dans une particule protéique composée des protéines lentivirales de structure et notamment de la glycoprotéine d'enveloppe.

Les lentivirus recombinants décrits dans la demande sont ainsi modifiés génétiquement de manière à ce que certains gènes constitutifs du virus infectieux natif soient supprimés et remplacés par une séquence d'acide nucléique d'intérêt à introduire dans les cellules cibles. Après fusion du virus à la membrane cellulaire, celui-ci injecte son acide nucléique dans la cellule qui, après réverse-transcription, peut s'intégrer dans le génome de la cellule hôte. Le matériel génétique ainsi transféré est ensuite transcrit et éventuellement traduit en protéines à l'intérieur de la cellule hôte.
Les lentivirus de l'invention peuvent être préparés à partir de différents sérotypes, notamment HIV-1, HIV2, SIV, FIV, BIV, VISNA, CAEV et EIAV. Des sérotypes particulièrement préférés sont le HIV, FIV et EIAV.

### Pseudotypage

Comme indiqué ci-avant, l'invention réside dans la mise en évidence des propriétés de ciblage de lentivirus pseudotypés avec des enveloppes particulières.
Le terme pseudotypage désigne un virus recombinant comportant une enveloppe différente de l'enveloppe sauvage, et possédant ainsi un tropisme modifié. Dans le cas des lentivirus pseudotypés, il s'agit de lentivirus possédant une enveloppe hétérologue d'origine non-lentivirale, par exemple provenant d'un autre virus, ou d'origine cellulaire.

Des vecteurs HIV-1 pseudotypés décrits dans l'art antérieur comportent la glycoprotéine d'enveloppe du Virus de la Stomatite Vésiculaire (VSV). Cette enveloppe présente des caractéristiques avantageuses telles que la résistance à l'ultracentrifugation et un très large tropisme. Contrairement à d'autres enveloppes comme celles des rétrovirus classiques (les rétrovirus amphotrope et ecotrope de MLV ou la gp120 du HIV mais aussi bien d'autres) la glycoprotéine de VSV n'est pas labile après ultracentrifugation. Ceci permet de concentrer les surnageants viraux et d'obtenir de hauts titres infectieux. Cette enveloppe confère par ailleurs aux virions un très large tropisme notamment *in vitro,* permettant l'infection de très nombreux types cellulaires. Le récepteur de cette enveloppe serait un motif phosphatidylsérine, présent à la surface de nombreuses cellules de différentes espèces.

Une caractéristique particulière décrite dans la demande consiste à utiliser une enveloppe (glycoprotéine) de lyssavirus et en particulier de virus du sérogroupe du virus de la Rage : Rabies (PV) (Tuffereau C., Benejean J., Blondel D., Kieffer B., Flamand A. ; Low-affinity nerve-growth factor receptor (P75NTR) can serve as a receptor for rabies virus ; EMBO J., 1998 Dec 15 ;17 (24) : 7250-9); Duvenhague (DUV), European bat type 1 (EB-1), European bat type 2 (EB-2), Lagos bat (LB), Mokola (MOK) (Bourhy H, Kissi B, Tordo N. Molecular diversity of the Lyssavirus genus. Virology. 1993 May;194(1):70-81.) (Badrane H, Bahloul C, Perrin P, Tordo N. Evidence of two Lyssavirus phylogroups with distinct pathogenicity and immunogenicity. J Virol. 2001 Apr,75(7):3268-76), Kotonkan (KOT), Obodhiang (OBD) et Rochambeau (RBU) (Della-Porta AJ, Brown F. The physico-chemical characterization of bovine ephemeral fever virus as a member of the family Rhabdoviridae. J Gen Virol. 1979 Jul; 44(1):99-112.) ou toute composition chimérique de ces enveloppes.

A la différence de VSV, la présente demande montre que les virus de la rage et de Mokola ont un tropisme chez l'animal très spécifique du système nerveux. Le système d'entrée de ces virus dépend en outre de récepteurs spécifiques. Pour la rage, plusieurs candidats sont proposés comme récepteurs : les récepteurs à l'acétylcholine (nicotinique en particulier), les molécules NCAM (140 et 180 kDa) ainsi que les récepteurs de basse affinité au NGF (p75).

Dans un mode de réalisation préféré, la demande décrit l'utilisation de vecteurs lentiviraux, par exemple de type HIV, pseudotypés avec une enveloppe de type PV (virus de la Rage) ou MOK (virus Mokola)
La demande montre en effet que ce type d'enveloppe permet un ciblage cellulaire, notamment le ciblage des cellules gliales de type astrocytaire.

Les pseudotypes MOK et PV peuvent en outre être ultracentrifugés tout comme VSV, et permettent donc la concentration des surnageants. Ils permettent en outre de manière surprenante l'obtention d'un tropisme restreint par rapport à VSV et donc le ciblage cellulaire *in vivo* dans le SNC. Il est également possible d'obtenir des clones stables sans système d'induction pour les glycoprotéines de PV et MOK. Ces clones stables sont utilisables pour le développement de lignées d'encapsidation stables permettant la production de vecteurs lentiviraux pseudotypés par l'une de ces deux enveloppes.

La demande décrit donc le pseudotypage de vecteurs lentiviraux par des enveloppes de lyssavirus (notamment PV et/ou MOK) pour transduire spécifiquement des astrocytes.
Elle décrit encore l'utilisation de ces vecteurs lentiviraux pseudotypés pour la préparation d'une composition destinée au transfert de gènes dans les astrocytes *in vivo.* Selon une caractéristique particulièrement avantageuse de l'invention, le lentivirus défectif utilisé est le virus de l'immunodéficience humaine. Selon une autre caractéristique particulièrement avantageuse le lentivirus utilisé comprend une ou plusieurs protéines virales sélectionnées parmi TAT et REV.

### Production de vecteurs lentiviraux pseudotypés recombinants

Les vecteurs lentiviraux décrits dans la demande peuvent être préparés de différentes manières, par transfection(s) transitoire(s), dans des lignées stables et/ou au moyen de virus helper.
La méthode décrite dans la demande prévoit, selon un mode particulièrement préféré, la combinaison d'un minimum de trois vecteurs pour produire un virion recombinant ou un rétrovirus recombinant.
Un premier vecteur fournit le vecteur lentiviral comportant les séquences virales agissant en *cis* et nécessaires au bon déroulement du cycle viral. De telles séquences incluent un ou plusieurs LTRs de lentivirus, une séquence Psi d'empaquetage d'origine lentivirale, des signaux de transcription inverse, un promoteur et/ou un enhancer et/ou des séquences de polyadénylation. Le premier vecteur contient également un site de clonage pour une séquence d'acide nucléique hétérologue à transférer dans une cellule non proliférative. Dans ce vecteur les LTR peuvent en outre être modifiés pour améliorer l'expression du transgène ou la sécurité du vecteur. Ainsi, il est possible de modifier par exemple la séquence du LTR 3' par suppression de la région U3 (WO 99/31251). Selon un mode préféré de réalisation de l'invention, il s'agit d'un plasmide vecteur comportant un génome lentiviral recombinant, de séquence LTR-psi-RRE-flap-Promoteur-transgene-LTR qui permet l'expression de l'ARN vecteur qui sera encapsidé dans les virions. Le transgène peut coder par exemple pour les facteurs trophiques suivants: CNTF, NGF, NT3, NT4, FGF, PDGF, etc., ou pour des enzymes restaurant une activité métabolique déficiente par exemple : TH, AADC, GTPC, B-glucuronidase, etc. Le transgène est typiquement placé sous contrôle d'un promoteur transcriptionnel, qui peut être homologue vis-à-vis du transgène ou hétérologue, par exemple un promoteur viral, cellulaire, synthétique, etc. Le promoteur utilisé peut être constitutif ou régulé, faible ou fort, spécifique de tissu ou ubiquitaire, etc. On utilise typiquement un promoteur viral tel que CMV, LTR, TK, etc ou un promoteur cellulaire tel que PGK, Rho, etc. Des promoteurs spécifiques de tissus peuvent être employés, même si les vecteurs de l'invention confèrent déjà un caractère de sélectivité d'expression.

Un deuxième vecteur, de trans-complémentation, fournit un acide nucléique codant une protéine lentivirale gag et une protéine lentivirale *pol*. Ces deux protéines sont dérivées d'un lentivirus et, de manière préférée, proviennent du VIH. Le deuxième vecteur est dépourvu de séquence d'encapsidation, de séquence codant pour une enveloppe, et, avantageusement, est également dépourvu de LTRs lentiviraux. De ce fait, les séquences codant pour des protéines *gag* et *pol* sont avantageusement placées sous contrôle d'un promoteur hétérologue, par exemple viral, cellulaire, etc., qui peut être constitutif ou régulé, faible ou fort. Il s'agit de préférence d'un plasmide transcomplémentant comportant une séquence CMV-Δpsi-gag-pol-Δenv-PolyA. Ce plasmide permet l'expression de toutes les protéines nécessaires à la formation de virions vides, exceptés les glycoprotéines d'enveloppe. Il est entendu que les gènes gag et pol peuvent aussi être portés par des plasmides différents.

Un troisième vecteur fournit un acide nucléique qui permet la production de la glycoprotéine d'enveloppe (env) choisie.
Cette enveloppe peut être choisie parmi les enveloppes citées précédemment, notamment une enveloppe de rhabdovirus, plus préférentiellement de lyssavirus, encore plus préférentiellement MOK ou PV. Ce vecteur est dépourvu de séquence d'encapsidation,de séquences codant gag ou pol et, avantageusement est également dépourvu de LTRs lentiviraux.
Avantageusement, les trois vecteurs utilisés ne comportent pas de séquence homologue suffisante pour permettre une recombinaison. Les acides nucléiques codant gag, *pol* et env peuvent être avantageusement des ADNc préparés selon les techniques conventionnelles, à partir de séquences des gènes viraux disponibles dans l'art antérieur et sur bases de données, ainsi qu'illustré dans les exemples.

Pour la production des virus recombinants défectifs pour la réplication, les vecteurs décrits ci-avant sont introduits dans des cellules compétentes et les virus fabriqués sont récoltés. Les cellules utilisées peuvent être toute cellule compétente, de préférence de mammifère, par exemple animale ou humaine, non pathogène. On peut citer par exemple les cellules 293, des cellules embryonnaires, des fibroblastes, des cellules musculaires, etc.
La demande décrit donc un procédé de préparation d'un lentivirus recombinant défectif pseudotypé, comprenant la transfection d'une population de cellules compétentes avec une combinaison de vecteurs tels que décrits ci-avant, et la récupération des virus produits.

La demande décrit ainsi un procédé particulièrement avantageux de production de lentivirus capables de cibler l'expression *in vivo* d'un transgène, dans des astrocytes humains, comprenant la transfection de cellules compétentes par :
a) un plasmide vecteur comportant une séquence LTR-psi-RRE-flap-Promoteur-transgene-LTR(ΔU3),
b) un plasmide transcomplémentant comportant une séquence CMV-Δpsi-gag-pol-Δenv-PolyA,
c) un plasmide d'enveloppe comportant une séquence CMV-env-PolyA, l'enveloppe étant une enveloppe du virus Mokola ou du virus de la rage.

Les lentivirus décrits dans la demande peuvent également être préparés à partir d'une lignée de cellules d'encapsidation produisant une ou plusieurs protéines *gag*, *pol* et *env*.
Selon un mode de réalisation particulier, on utilise une lignée cellulaire exprimant la protéine env de manière stable (c'est-à-dire à partir d'une séquence intégrée dans son génome). En effet, contrairement aux enveloppes lentivirales, les enveloppes MOK ou PV par exemple ne présentent pas de toxicité significative pour les cellules et peuvent être exprimées de manière stable et constitutive (ou régulée) dans une lignée.

De ce fait, dans un mode particulier de mise en oeuvre, le procédé décrit dans la demande comprend la transfection de deux vecteurs seulement (le vecteur lentiviral et le vecteur de transcomplémentation) dans une lignée de cellules exprimant la protéine env choisie. Les cellules utilisées pour la préparation d'une telle lignée sont, par exemple, les cellules compétentes mentionnées ci-avant. Une telle lignée est également décrite dans la présente demande.

Selon un autre mode de réalisation, la lignée utilisée exprime également les protéines *gag* et *pol* lentivirales. Dans ce cas, le procédé comprend simplement la transfection du vecteur lentiviral.

A cet égard, il est possible d'utiliser une cellule lentivirale d'encapsidation telle celle décrite dans la demande de brevet WO 99/58701, dans laquelle les gènes gag et pol du HIV sont présents sur des constructions séparées.

De manière préférée, les lentivirus produits sont dérivés du virus HIV-1, HIV-2 SIV, FIV,BIV, VISNA, CAEV ou EIAV.

De manière surprenante, la présente demande montre que les vecteurs lentiviraux pseudotypés ainsi obtenus sont capables d'infecter préférentiellement certaines sous-populations de cellules nerveuses, en particulier les astrocytes *in vivo.* Par « préférentiellement », il faut entendre que les lentivirus selon l'invention ciblent essentiellement les astrocytes mais sont néanmoins capables de transfecter d'autres types cellulaires tels que d'autres cellules gliales ou des neurones. D'autres sous-populations de cellules nerveuses qui peuvent être ciblées par des vecteurs décrits dans la demande sont par exemple des cellules microgliales, des cellules endothéliales ou des oligodendrocytes. Dans une application particulière concernant le transfert de gènes dans l'oeil, les vecteurs décrits dans la demande pseudotypés avec l'enveloppe Mokola permettent un transfert sélectif vers les cellules de l'épithélium pigmentaire.

Les lentivirus décrits dans la demande peuvent enfin être utilisés pour la fabrication d'une composition pharmaceutique destinée à traiter une maladie du système nerveux ou une maladie neurodégénérative et notamment la maladie d'Alzheimer, la maladie de Parkinson, la Chorée de Huntington, les SLA ou SMA, des dégénérescences oculaires ou encore les traumas du système nerveux central (attaque cérébrale, épilepsie, lésions de la moelle épinière, etc.), les maladies affectant le système nerveux central (MPS, etc.), les glioblastomes ou astrocytomes, ou les maladies métaboliques affectant le système nerveux (mucopolysacharidoses, Charcot-Marie, etc.).

Un autre objet décrit dans la demande concerne l'utilisation combinée de plusieurs lentivirus pseudotypés avec une enveloppe de lyssavirus différente, en vue de transférer et d'exprimer un acide nucléique dans les cellules du système nerveux. L'utilisation combinée peut comprendre des administrations séquentielles des différents virus, ou une administration simultanée.

Comme indiqué ci-avant, les vecteurs décrits dans la demande peuvent permettre le transport et l'expression de multiples acides nucléiques dans les cellules nerveuses, comme par exemple des acides nucléiques catalytiques (antisens, ribozymes, etc.), des acides nucléiques codant pour des facteurs de croissance, des facteurs trophiques, des cytokines, des facteurs de stimulation des colonies, des agents anticancéreux, des toxines, des enzymes, des neurotransmetteurs ou leurs précurseurs, etc.

La composition pharmaceutique contenant le lentivirus décrit dans la demande peut être administrée à un patient par voie intracérébrale ou systémique compte-tenu du tropisme particulier des vecteurs lentiviraux pseudotypés notamment pour les astrocytes. Ainsi, il peut s'agir d'une administration par voie intracérébrale, intra-striatale, intra-veineuse, intra-artérielle, dans l'espace sous-rétinien, etc. Des modes d'injection préféré sont l'injection intra-cérébrale et l'injection dans l'espace sous-rétinien. Pour l'utilisation pour le transfert de gènes dans l'oeil, on préfère tout particulièrement des virus pseudotypés avec une enveloppe Mokola.

La composition est administrée avantageusement à raison de 10² à 10⁹ particules par dose, typiquement de 10³ à 10⁸. Les lentivirus peuvent être conditionnés dans toute solution adaptée, telle que solution saline, isotonique, tamponnée, éventuellement associée à des agents stabilisants tels que de l'albumine isogénique ou tout autre protéine stabilisante, du glycérol, etc., ainsi que des facteurs adjuvants comme le polybrène ou le DEAE dextran, etc.

D'autres avantages de l'invention sont illustrés plus en détails dans les exemples qui suivent, qui doivent être considérés comme illustratifs.

### Légende des figures

Figure 1 : Intensité moyenne de la GFP dans les cellules transduites.
Figure 2 : Pourcentage de cellules transduites.

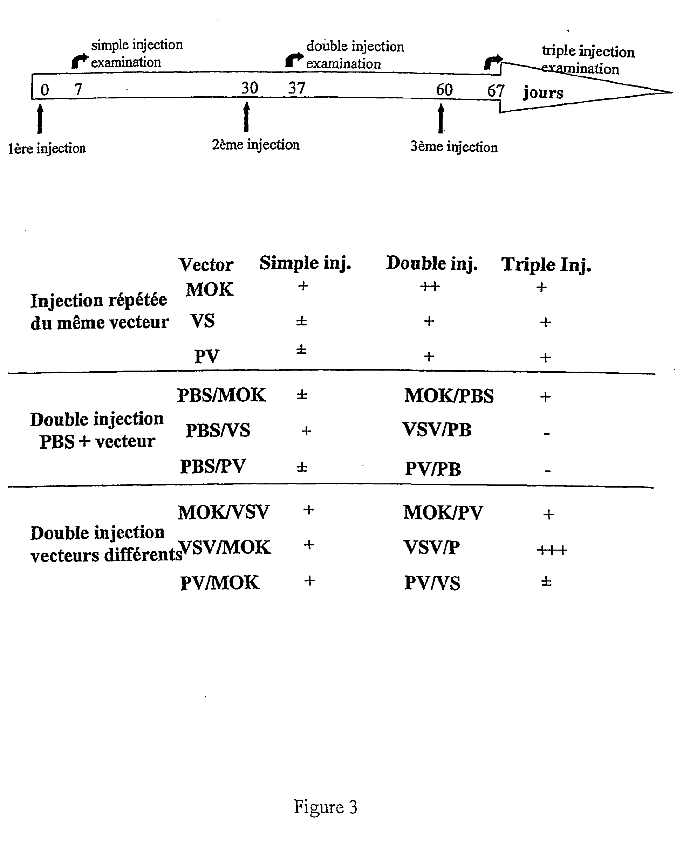

Figure 3 : Déroulement de l'expérience - Réponse immunitaire (CD4/CD8)
Figure 4: Angiographie des globes oculaires injectés par voie sous-rétinienne avec un vecteur dérivé de HIV-1 et exprimant la GFP sous le contrôle du promoteur CMV. A gauche apparaît le vecteur pseudotypé par la protéine VSV-G et à droite le vecteur pseudotypé par la protéine Mokola-G.
Figure 5 : Epithélium pigmentaire des globes oculaires injectés par voie sous-rétinienne avec une vecteur dérivé de HIV-1 et exprimant la GFP sous le contrôle du promoteur CMV. (A) vue d'ensemble de la zone tranduite). (B) vecteur pseudotypé par la protéine VSV-G ; (C) vecteur pseudotypé par la protéine Mokola-G. Dans la zone transduite, 100% des cellules expriment la GFP. L'aspect hexagonal et binucléé des cellules permet d'affirmer qu'il s'agit bien de l'épithélium pigmentaire.
Figure 6 : Immunohistochimie anti-GFP sur coupes fines de rétine. Avec le vecteur pseudotypé par VSV-G (A) des cellules marquées sont détectées dans la couche des photorécepteurs (flèches). Avec le vecteur pseudotypé par Mokola-G (B), seules les cellules de l'épithélium pigmentaire (ep) sont marquées.

### Exemples

### 1. Production des virus pseudotypés (VSV, MOK ET PV)

Les vecteurs de séquence LTR-Ψ-RRE-flap-CMV GFP-LTR ou LTR-Ψ-RRE-flap-PGK-GFP-LTR(ΔU3), le plasmide de transcomplémentation Δ8.71 et les plasmides d'enveloppe CMV-VSV-G, CMV-MOK-G ou CMV-Rab-G ont été utilisés pour produire les virus pseudotypés.

Au jour 0, des boîtes de 160 cm² remplies de cellules 293T à faible confluence (environ 50%), sont préparées.
Au jour 1, le milieu de culture est remplacé par du milieu frais et la transfection est opérée avec les trois plasmides.
Au jour 2, le milieu est à nouveau changé.
Au jour 3, le surnageant est récolté puis traité à la DNAse pendant 15 min. à 37°C. Il est ensuite centrifugé à 3000 rpm pendant 5 min. pour éliminer les déchets cellulaires et ultracentrifugé à 4°C pendant 1h30min (rotor SW28) puis resuspendu en PBS 1X. Les résidus sont ensuite éliminés par courtes centrifugations à 8000 rpm pendant 30 secondes.
Le surnageant ainsi purifié est ensuite aliquoté et stocké à -80C.

La titration des stocks se fait par dosage ELISA de la protéine de capside (p24). Elle est exprimée en nanogrammes de molécules p24 par microlitre de surnageant.

| | |
|---|---|
| Stocks #1: | CMV-GFP |
| VSV: | 78 ng p24/µl |
| MOK: | 19 ng p24/µl |
| PV : | 29 ng p24/µl |

| | |
|---|---|
| Stocks #2: | CMV-GFP |
| VSV: | 89 ng p24/µl |
| MOK : | 30 ng p24/µl |
| PV : | 64 ng p24/µl |

| | |
|---|---|
| Stocks #3: | PGK-GFP |
| VSV : | 30 ng p24/µl |
| MOK : | 56 ng p24/µl |
| PV : | 21 ng p24/µl |

### 2. Transduction de lignées cellulaires in vitro par les virus pseudotypés

La transduction de cellules nerveuses a été testée sur 7 lignées différentes avec les vecteurs contenant une cassette CMV-GFP. Les lignées testées sont les suivantes :
- CHP212 et SKNSH : neuroblastomes humains
- PC12 : phéochromocytome de rat
- Cath.a et Cath.b : lignées centrales cathécolaminergiques de souris transformées par SV40
- Path.1 et Path.2 : lignées périphériques catécholaminergiques de souris transformées par SV40

Une totalité de 100.000 cellules sont déposées dans les puits d'une plaque de 24 puits. Le lendemain les cellules sont infectées avec du milieu de culture contenant 20 ng de p24 de vecteur dans 200 µl final. Les cellules sont incubées avec le virus toute la nuit. Le lendemain le milieu est changé et remplacé par un milieu frais sans virus. Les cellules sont dissociées et fixées pour analyse en cytométrie de flux (FACS), 48h après le retrait des virus.

Les cellules SKNSH, CHP212 et PC12 ont été infectées avec les stocks#1. Les cellules Cath et Path avec les stocks#2.

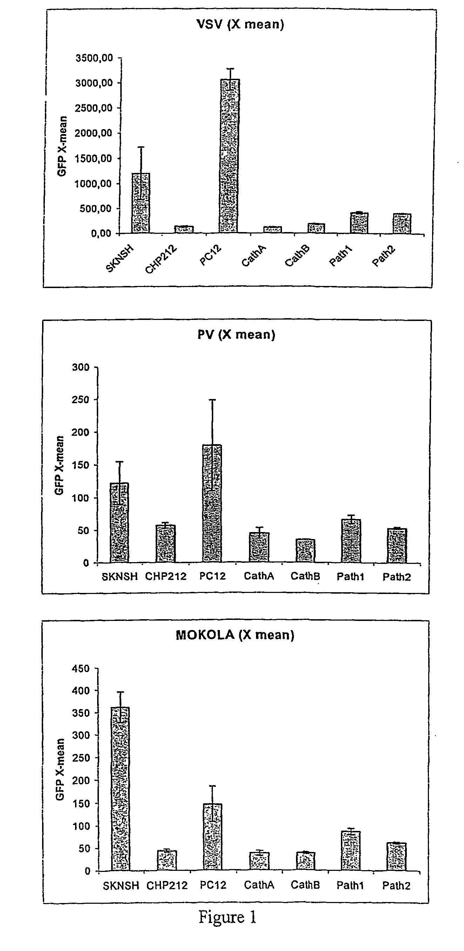

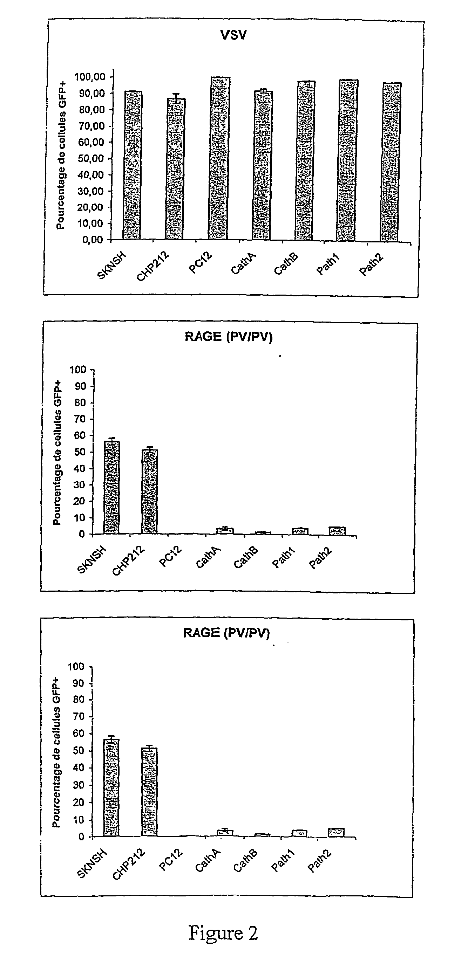

Les figures 1A, 1 B, 1C et 2A, 2B, 2C mettent en évidence une transduction très efficace, par les pseudotypes VSV, de toutes les lignées testées (en pourcentage). Les variations d'intensité traduisent l'activité relative du promoteur CMV dans les différentes lignées testées (GFP X-mean).

En revanche, les pseudotypes PV ne transduisent efficacement (sup. à 10%) que les lignées humaines CHP212 et SKNSH.
Les pseudotypes MOK transduisent efficacement les lignées CHP212, SKSNH, Cath.b, Path.1 et Path.2.

Ainsi il apparaît clairement que les pseudotypes MOK et PV présentent une restriction d'hôte inexistante chez VSV dans les lignées testées.

### 3. Transduction in vivo de cerveau de souris C57B16

Pour chaque pseudotype, 3 souris C57B16 (8 semaines) ont subi une injection dans le striatum. Une dose de 20 ng de p24 (vecteurs CMV-GFP) a été injectée dans un volume de 2 microlitres. Les coordonnées stéréotaxiques utilisées par rapport au lambda sont: A: +4,6 ; L: +2,2 ; V1: -3,5 (1µl) et V2: -3,3 (1µl). La vitesse d'injection est de 0,25 µl/min.

Une semaine après l'injection, les souris sont sacrifiées. Les cerveaux de deux souris sont perfusés (PFA 4%) et le troisième est congelé sans perfusion. Les cerveaux sont coupés au cryostat dans la zone d'injection (20µm d'épaisseur).

L'expression de la GFP peut être visualisée directement sous microscope à fluorescence. Le phénotype des cellules transduites est déterminé par immunomarquage des coupes avec un anticorps anti-GFAP pour les astrocytes, par un anticorps anti-NeuN pour les neurones et par un anticorps anti-CNPase pour les oligodendrocytes.

L'analyse de la co-expression de la GFP et des différents marqueurs utilisés est faite sur microscope confocal.

Les résultats obtenus montrent que les pseudotypes VSV transduisent différentes populations cellulaires, dont les astrocytes et les neurones. Dans le cas des lentivirus pseudotypés MOK, en revanche, on transduit spécifiquement des astrocytes. De très rares neurones semblent être transduits. De même, pour les pseudotypes PV, seuls les astrocytes semblent être transduits.

D'autre part, la quantité de cellules exprimant la GFP est très importante avec les pseudotypes VSV et MOK (niveaux comparables), même si le nombre de cellules transduites avec les pseudotypes PV semble plus faible.

Pour confirmer ces résultats de ciblage préférentiel des astrocytes avec les pseudotypes utilisant MOK et PV, des injections ont été réalisées dans des conditions similaires avec les vecteurs PGK-GFP. Dans cette expérience une dose de 60ng de p24 de pseudotypes VSV et MOK a été injectée dans des cerveaux de souris C57B16.
Les expériences de double marquage avec un marqueur de neurone (NeuN) et un marqueur d'astrocytes (GFAP) montrent un tropisme uniquement astrocytaire pour les pseudotypes MOK, alors que de nombreux neurones sont transduits avec les pseudotypes VSV, confirmant ainsi la spécificité d'infection des astrocytes avec MOK.

**Tableau récapitulatif des résultats de phénotypage:**

| | NeuN | GFAP | CNPase |
|---|---|---|---|
| VSV - CMV | + | +++ | - |
| MOK - CMV | - | ++++ | - |
| PV - CMV | - | + | - |
| VSV - PGK | +++ | + | nd |
| MOK - PGK | - | +++ | nd |
| PV - PGK | - | - | nd |

### 4. Etude de la réaction immunitaire (injections multiples)

Cette expérience a été réalisée dans le but d'étudier la possibilité de faire des injections répétées avec les différents pseudotypes. Des vecteurs pseudotypés avec la même glycoprotéine ont été injectés à 2 ou 3 reprises dans le cerveau de souris C57BI6. La combinaison d'injections répétées avec deux pseudotypes différents a aussi été réalisée afin de tester les réactions immunitaires croisées entre les différents pseudotypes.
L'expression de la GFP est visualisée directement par fluorescence. Dans tous les cas il est possible de repérer des cellules GFP+ dans le site de la dernière injection, signe d'une faible réaction immunitaire.
Une étude plus détaillée de la réaction CTL (réaction cellulaire des lymphocytes T) a été réalisée par des immunomarquages dirigés contre les antigènes CD4 et CD8.
Lorsqu'une seule injection est réalisée, la réaction CTL (CD4 et CD8) est très faible, c'est-à-dire quasiment nulle au niveau des animaux contrôles n'ayant reçu que de la solution saline.

Lorsque deux injections successives sont réalisées, la réaction observée est très modérée (CD4 et CD8 supérieurs au PBS) mais très inférieure à celle provoquée par une injection simple d'adénovirus par exemple.

Lorsque trois injections successives sont réalisées, la réaction CD4 et CD8 est relativement importante. Les vecteurs ne sont cependant pas entièrement neutralisés puisque l'on retrouve une expression au niveau du site de la troisième injection.
La réaction CD4/CD8 (lymphocytes T) peut être provoquée soit par le vecteur (la glycoprotéine d'enveloppe en particulier) soit par l'expression du transgène GFP.

Ces expériences confirment l'absence de réaction immunitaire *in vivo* après plusieurs injections, et démontrent la possibilité de réaliser de telles injections répétées dans des approches thérapeutiques ou expérimentales.

### 5. Tropisme des vecteurs lentiviraux dérivés de HIV-1 et pseudotypés par VSV- ou Mokola-G pour les cellules rétiniennes

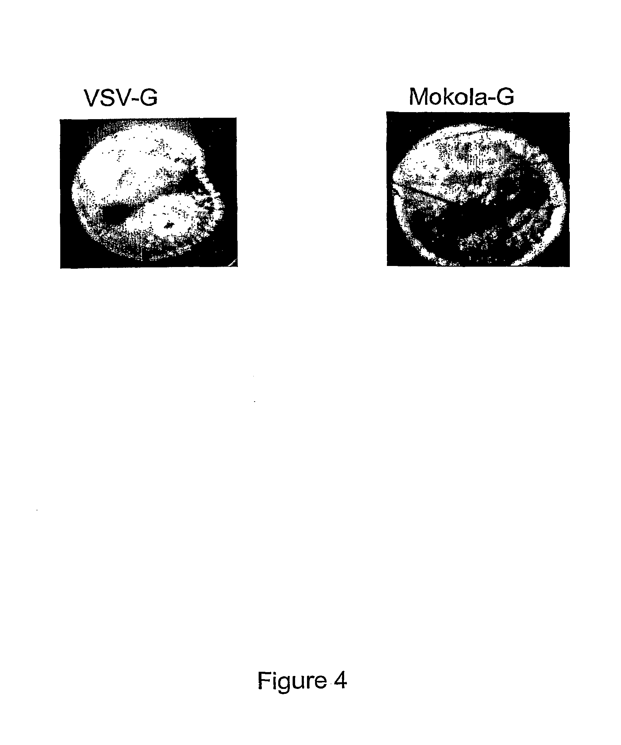

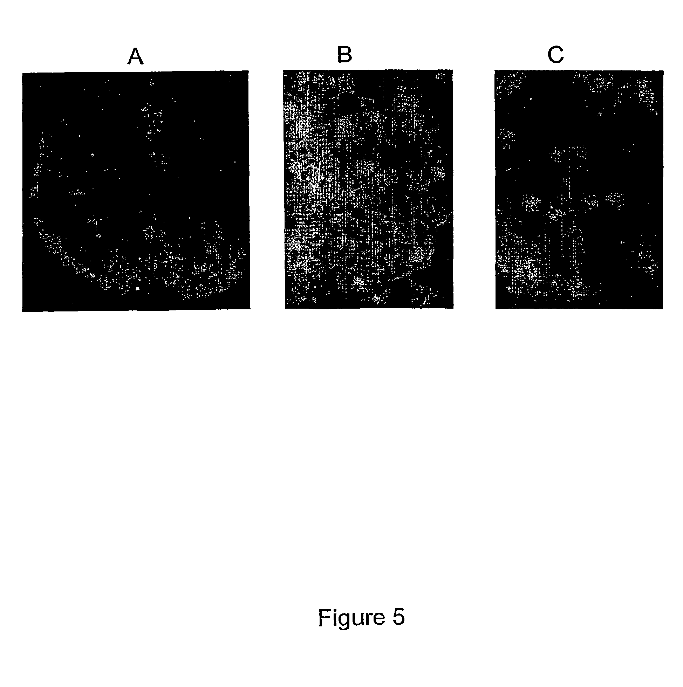

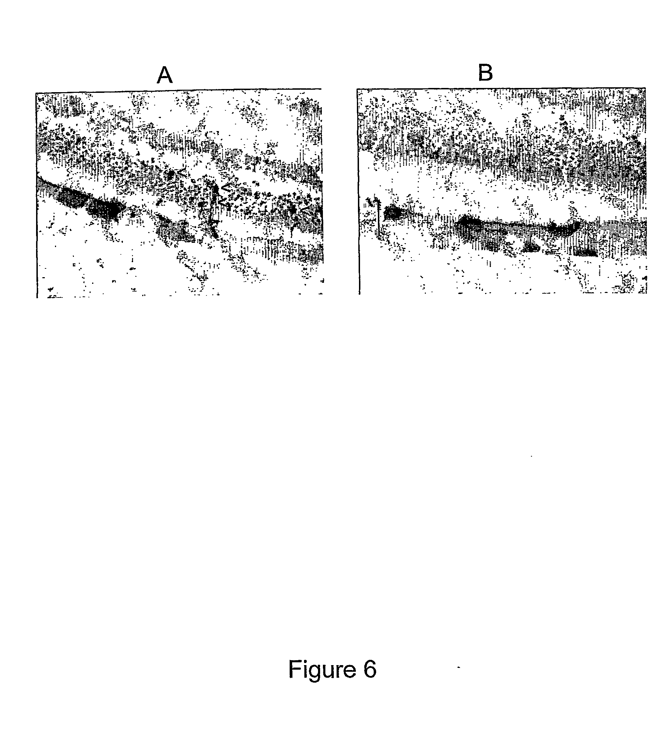

Les vecteurs lentiviraux dérivés de HIV-1 et pseudotypés par VSV- ou Mokola-G ont été injectés dans l'espace sous-rétinien du rat adulte (souche rdy). Pour chaque pseudotype, différents promoteurs permettant l'expression du gène rapporteur Green Fluorescent Protein (GFP) ont été testés : le promoteur CMV (promoteur viral ubiquitaire), le promoteur PGK (promoteur ubiquitaire du gène de la phosphoglycérate kinase de souris) et le promoteur Rho (fragment de 2,2 kilobases du promoteur du gène de la rhodopsine, une protéine exprimée spécifiquement dans les cellules de la rétine). Une semaine et trois semaines après injections, la GFP est détectée sur l'animal vivant par angiographie. A 4 semaines, l'animal est sacrifié et la GFP est visualisée de 2 manières différentes : directement, sur les épithéliums pigmentaires de la rétine montés à plat, ou par immunohistochimie sur des coupes fines de rétine. Ces expériences ont permis de dégager les résultats suivants :
1. Les deux pseudotypes permettent une expression forte de GFP au niveau de la rétine qui, avec le promoteur CMV est détectable *in vivo* par angiographie (figure 4).
2. Les deux pseudotypes permettent de tranduire les cellules de l'épithélium pigmentaire avec une grande efficacité. Environ un quart à un tiers de la surface de l'épithélium exprime la GFP. Dans le zone transduite, 100 % des cellules expriment la GFP. Cela apparaît clairement sur les épithéliums pigmentaires montés à plat (figure 5).
3. Par immunohistochimie, l'expression de GFP est détectée au niveau des photorécepteurs après injection sous-rétinienne des vecteurs pseudotypés par la protéine VSV-G (figure 6a). Cette expression est plus importante avec le promoteur spécifique Rho qu'avec les promoteurs ubiquitaires CMV et PGK. Par opposition, après injection des vecteurs pseudotypés par la protéine Mokola-G, aucune expression de GFP n'est détectée au niveau de la couche des photorécepteurs, et ce quelque soit le promoteur utilisé pour contrôler l'expression de la GFP (figure 6b).

Ces expériences démontrent que les vecteurs lentiviraux dérivés de HIV-1 et pseudotypés par l'enveloppe Mokola G permettent de transduire spécifiquement les cellules de l'épithélium pigmentaire de la rétine à la suite d'une injection sous-rétinienne.

## Revendications

1. Utilisation d'un lentivirus défectif pseudotypé par une enveloppe de virus Mokola pour la préparation d'une composition destinée au transfert sélectif de gènes dans les astrocytes du système nerveux central d'un sujet *in vivo.*

2. Utilisation selon la revendication 1, **caractérisée en ce que** le gène est un acide nucléique catalytique ou un acide nucléique codant un facteur de croissance, un facteur trophique, une cytokine, un facteur de stimulation des colonies, un agent anticancéreux, une toxine, un enzyme ou un neurotransmetteur ou son précurseur.

3. Utilisation selon l'une des revendications 1 ou 2, **caractérisée en ce que** le lentivirus défectif pseudotypé comporte un génome lentiviral comprenant une séquence LTR-Psi-RRE-flap-Promoteur-Transgène-LTR.

4. Utilisation d'un lentivirus selon l'une des revendications 1 à 3, **caractérisé en ce qu'**il s'agit d'un virus de type virus de l'immunodéficience humaine de type 1 (HIV-1), virus de l'immunodéficience humaine de type 2 (HIV-2), virus de l'immunodéficience simienne (SIV), virus de l'immunodéficience féline (FIV), virus de l'anémie infectieuse équine (EIAV), virus de l'immunodéficience bovine (BIV), virus du VISNA-Maedi (VISNA) ou virus de l'arthrite-encéphalite caprine (CAEV).

5. Utilisation d'un lentivirus pseudotypé selon l'une des revendications 1 à 4, pour la fabrication d'une composition thérapeutique destinée à traiter une maladie du système nerveux ou une maladie affectant le système nerveux.

6. Utilisation selon la revendication 5, pour le traitement des maladies neurodégénératives.

7. Utilisation selon la revendication 6, dans laquelle la maladie neurodégénérative est choisie parmi la maladie de Parkinson, la maladie de Huntington, la maladie d'Alzheimer, le SLA et le SMA.

8. Utilisation selon la revendication 5, pour le traitement des traumas du système nerveux central.

9. Utilisation selon la revendication 8, dans laquelle le trauma est choisi parmi le trauma de la moelle épinière et l'attaque cérébrale.

10. Utilisation selon la revendication 5, pour le traitement de maladies métaboliques affectant le système nerveux.

11. Utilisation selon la revendication 10, dans laquelle la maladie métabolique est choisie parmi les mucopolysaccharidoses et la maladie de Charcot-Marie.

## Claims

1. Use of a defective lentivirus pseudotyped with a Mokola virus envelope, for preparing a composition intended for the selective transfer of genes into the astrocytes of the central nervous system of an individual *in vivo.*

2. The use according to claim 1, **characterized in that** the gene is a catalytic nucleic acid or a nucleic acid encoding a growth factor, a trophic factor, a cytokine, a colony stimulating factor, an anticancer agent, a toxin, an enzyme, or a neurotransmitter or its precursor.

3. The use according to one of claims 1 or 2, **characterized in that** the pseudotyped defective lentivirus comprises a lentiviral genome comprising a sequence LTR-Psi-RRE-flap-Promoter-Transgene-LTR.

4. The use of a lentivirus according to one of claims 1 to 3, **characterized in that** it is a virus of the type 1 human immunodeficiency virus (HIV-1), the type 2 human immunodeficiency virus (HIV-2), the simian immunodeficiency virus (SIV), the feline immunodeficiency virus (FIV), the equine infectious anemia virus (EIAV), the bovine immunodeficiency virus (BIV), the VISNA-Maedi virus(VISNA) or the caprine arthritis-encephalitis virus (CAEV) type.

5. The use of a pseudotyped lentivirus according to one of claims 1 to 4, for producing a therapeutic composition intended for treating a disease of the nervous system or a disease affecting the nervous system.

6. The use according to claim 5, for treating neurodegenerative diseases.

7. The use according to claim 6, wherein the neurodegenerative disease is selected from Parkinson's disease, Huntington's disease, Alzheimer's disease, SLA and SMA.

8. The use according to claim 5, for treating traumas of the central nervous system.

9. The use according to claim 8, wherein the trauma is selected from the spinal cord trauma and the cerebral attack.

10. The use according to claim 5, for treating metabolic diseases affecting the nervous system.

11. The use according to claim 10, wherein the metabolic disease is selected from mucopolysaccharidosis and the Charcot-Marie-Tooth disease.

## Patentansprüche

1. Verwendung eines durch eine Mokola-Virushülle pseudotypisierten unvollständigen (defekten) Lentivirus für die Herstellung einer Zusammensetzung, die bestimmt ist für die selektive in vivo-Genübertragung in Astrozyten des Zentralnervensystems eines Individuums.

2. Verwendung nach Anspruch 1, **dadurch gekennzeichnet, dass** das Gen eine katalytische Nucleinsäure oder eine Nucleinsäure ist, die einen Wachstumsfaktor, einen trophischen Faktor, ein Cytokin, einen Kolonien-Stimulierungs-Faktor, ein Antikrebsmittel, ein Toxin, ein Enzym oder einen Neurotransmitter oder einen Vorläufer davon codiert.

3. Verwendung nach einem der Ansprüche 1 oder 2, **dadurch gekennzeichnet, dass** das pseudotypisierte unvollständige (defekte) Lentivirus ein Lentivirus-Genom umfasst, das eine LTR-Psi-RRE-flap-Promotor-Transgen-LTR-Sequenz umfasst.

4. Verwendung eines Lentivirus nach einem der Ansprüche 1 bis 3, **dadurch gekennzeichnet, dass** es sich dabei handelt um ein Human-Immundefizit-Virus vom Typ 1 (HIV-1), ein Human-Immundefizit-Virus vom Typ 2 (HIV-2), ein Affen-Immundefizit-Virus (SIV), ein Katzen-Immundefizit-Virus (FIV), ein Pferde-Infekt-Anämie-Virus (EIAV), ein Rinder-Immundefizit-Virus (BIV), ein VISNA-Maedl-Virus (VISNA) oder ein Ziegen-Arthritis-Enzephalitis-Virus (CAEV).

5. Verwendung eines pseudotypisierten Lentivirus nach einem der Ansprüche 1 bis 4 zur Herstellung einer therapeutischen Zusammensetzung, die bestimmt ist für die Behandlung einer Erkrankung des Zentralnervensystems oder einer Erkrankung, die das Zentralnervensystem befällt.

6. Verwendung nach Anspruch 5 für die Behandlung von neurodegenerativen Erkrankungen.

7. Verwendung nach Anspruch 6, in der die neurodegenerative Erkrankung ausgewählt ist aus der Gruppe Parkinson-Krankheit, Huntington-Krankheit, Alzheimer-Krankheit, SLA und SMA.

8. Verwendung nach Anspruch 5 für die Behandlung von Traumata des Zentralnervensystems.

9. Verwendung nach Anspruch 8, bei der das Trauma ausgewählt wird aus der Gruppe Rückenmarkstrauma und Schlaganfall (Gehirnschlag).

10. Verwendung nach Anspruch 5 für die Behandlung von Stoffwechsel-Erkrankungen, die das Zentralnervensystem befallen.

11. Verwendung nach Anspruch 10, bei der die Stoffwechsel-Erkrankung ausgewählt wird aus der Gruppe der Mucopolysaccharidosen und der Charcot-Marie-Krankheit.
